# EUROPÄISCHE PATENTSCHRIFT

(11) **EP 1 793 743 B1**
(45) Veröffentlichungstag und Bekanntmachung des Hinweises auf die Patenterteilung: **18.11.2009**
(21) Anmeldenummer: 04765482.7
(22) Anmeldetag: 22.09.2004
(51) Int. Cl.: A61B 17/12

(54) **VORRICHTUNG ZUR IMPLANTATION VON MIKROWENDELN**
MICRO-SPIRAL IMPLANTATION DEVICE
DISPOSITIF D'IMPLANTATION DE MICRO-HELICES

(43) Veröffentlichungstag der Anmeldung: 13.06.2007
(73) Patentinhaber: Dendron GmbH, 44799 Bochum (DE)
(72) Erfinder: MONSTADT, Hermann, 44797 Bochum (DE); FLESSER, Achim, 40822 Mettnam (DE); HANNES, Ralf, 44137 Dortmund (DE)
(74) Vertreter: Behrendt, Arne
(86) Internationale Anmeldenummer: PCT/EP2004/010612
(87) Internationale Veröffentlichungsnummer: WO 2006/032291

(56) Entgegenhaltungen:
- EP-A- 0 792 623
- WO-A-99/51151
- US-A- 5 382 260
- US-A1- 2004 002 733

## Beschreibung

Die Erfindung betrifft eine Vorrichtung zur Implantation von Mikrowendeln in Körperhohlräumen und Blutgefäßen, insbesondere Aneurysmen, wobei die Mikrowendeln aus einer Vielzahl von Windungen bestehende Drähte sind, mindestens eine Mikrowendel als Okklusionswendel dem Verschließen des Körperhohlraumes oder Blutgefäßes dient und die Vorrichtung aus einem Katheter, einer oder mehreren im Katheter in Längsrichtung verschiebbaren Mikrowendeln und mindestens einem das Lumen der Okklusionswendel zumindest teilweise durchlaufenden Sicherungsmittel besteht und das Sicherungsmittel in seinen Endbereichen in den Mikrowendeln festgelegt ist. Die Erfindung bezieht sich weiterhin auf eine Okklusionswendel zur Verwendung in Verbindung mit der vorgenannten Vorrichtung.

Der Einsatz endovaskulärer Techniken zur Okklusion von Körperhohlräumen oder Gefäßen wie Arterien, Venen, Eileitern oder vaskulären Fehlbildungen (z. B. vaskulärer Aneurysmen) ist bekannter Stand der Technik. Die Okklusionswendel wird dabei in der Regel mit Hilfe eines endovaskulären Führungsdrahtes durch einen Katheter in den zu okkludierenden Hohlraum eingeführt und deponiert.

Im Vorfeld der Deponierung werden die zu implantierenden Okklusionswendeln mit Hilfe des Katheters durch das Blutgefäßsystem gesteuert und am Zielort aus dem Katheter in den zu okkludierenden Hohlraum vorgeschoben. Im Idealfalle schließt sich die Abtrennung der Wendel an. Im Falle fehlerhafter Positionierung oder einer für den zu okkludierenden Bereich zu groß dimensionierten Okklusionswendel muß diese jedoch repositioniert oder vollständig in den Katheter zurückgezogen werden, um anschließend eine korrekte Positionierung oder die Einbringung einer korrekt dimensionierten Okklusionswendel zu ermöglichen. Derartige Manöver im Gefäßsystem bergen die Gefahr, daß Teile der Wendel durch Zug- oder Torsionsbeanspruchung auseinandergezogen und so irreversibel plastisch deformiert werden, reißen oder brechen, was eine lebensgefährliche Embolie nach sich ziehen kann.

Um diese Gefahren zu minimieren, ist es u. a. aus der europäischen Patentschrift EP 0 792 623 B1 bekannt, durch das Lumen der Okklusionswendel ein polymeres, ausdehnungswiderstandsfähiges Element verlaufen zu lassen, das an mindestens zwei Stellen der Okklusionswendel fest angebracht ist. Eine solche Konstruktion macht es möglich, eine Okklusionswendel zu repositionieren oder in den Katheter zurückzuziehen, ohne dabei die Okklusionswendel auseinanderzuziehen und gegebenenfalls irreversibel zu deformieren.

Dokument US 2004/002733 A1 offenbart eine Vorrichtung zur Implantation von Mikrowendlen in Körperhohlräumen nach dem Oberbegriff des Anspruchs 1.

Nachteilig macht sich jedoch bei diesem Stand der Technik bemerkbar, daß bei Anwendung einer zu hohen Rückzugskraft die Gefahr des plötzlichen Reißens des Polymerfadens besteht. In einem solchen Fall wirkt die gesamte Zugbelastung plötzlich auf die Okklusionswendel selbst, was in einer Deformierung oder gar im Zerreißen der Okklusionswendel resultieren kann. Aufgrund der Plötzlichkeit des Reißens des Polymerfadens können die Auswirkungen im Blutgefäß beträchtlich sein und sind praktisch nicht zu kontrollieren.

Angesichts der vorbeschriebenen Probleme stellt sich daher die Aufgabe, eine Vorrichtung zur Implantation von Okklusionswendeln zur Verfügung zu stellen, die das Einbringen der Okklusionswendeln mit im Vergleich zum Stand der Technik erhöhter Sicherheit für den Patienten erlaubt.

Diese Aufgabe wird erfindungsgemäß durch eine Vorrichtung der eingangs genannten Art gelöst, bei der das Sicherungsmittel zumindest in einem Endbereich zur Herstellung einer reibschlüssigen Verbindung so in einer Mikrowendel festgelegt ist, das diese Verbindung durch Überschreiten einer bestimmten auf das Sicherungsmittel wirkenden Zugkraft von der Mikrowendel lösbar ist.

Das Vorsehen einer nur reibschlüssigen, nicht jedoch festen Verbindung in einem Endbereich des Sicherungsmittels gewährleistet, daß das Sicherungsmittel in diesem Endbereich nur durch Reibungskräfte in der entsprechenden Mikrowendel festgelegt ist. Durch die genaue Ausgestaltung der reibschlüssigen Verbindung, die für den Fachmann ohne weiteres möglich ist, lassen sich diese Reibungskräfte so einstellen, daß die Kraft kleiner ist, als die Zugkraft, die auf das Sicherungsmittel einwirken muß, um es zum Reißen zu bringen. Auf der anderen Seite muß die Reibungskraft so hoch eingestellt werden, daß im Normalfall das Zurückziehen und Repositionieren der Okklusionswendel problemlos möglich ist. Für den Fall, daß die Zugkräfte so stark ansteigen, daß das Reißen des Sicherungsmittels zu erwarten wäre, zieht sich das Sicherungsmittel an der Stelle seiner Festlegung in der Mikrowendel aus dieser heraus, so daß die reibschlüssige Verbindung gelöst und das Reißen des Sicherungsmittels verhindert wird. Dieser Prozeß der Lösung der reibschlüssigen Verbindung erfolgt nicht plötzlich wie das Reißen des Sicherungsmittels sondern sukzessive, so daß keine plötzlich freiwerdenden Kräfte auftreten, die die bei den Ausführungen zum Stand der Technik beschriebenen negativen Auswirkungen zur Folge haben können. Durch die erfindungsgemäße Ausbildung einer Implantationsvorrichtung wird ein "Rutschkupplungseffekt" hervorgerufen.

Die reibschlüssige Verbindung des Sicherungsmittels mit einer Mikrowendel kann auf verschiedene Art und Weise hergestellt werden. Eine Möglichkeit besteht darin, das Sicherungsmittel zwischen einzelnen oder mehreren Windungen einer Mikrowendel so verlaufen zu lassen, daß es zwischen diesen Windungen eingeklemmt ist. Das Sicherungsmittel verläuft dabei durch mehrere Lücken zwischen den Windungen einer Mikrowendel. Die Stärke der reibschlüssigen Verbindung kann dabei durch die Zahl der Einpressungen des Sicherungsmittels in die Windungslücken eingestellt werden. Das Sicherungsmittel kann dabei sowohl zwischen Windungen auf gegenüberliegenden Seiten der Mikrowendel eingeklemmt sein, so daß das Sicherungsmittel das Lumen der Mikrowendel in diesem Endbereich mehrfach kreuzt, als auch durch die Windungen der Mikrowendel auf einer Seite verlaufen. Möglich ist es auch, daß das Sicherungsmittel in seinem Endbereich zur Herstellung der reibschlüssigen Verbindung um den die Windungen bildenden Draht der Mikrowendel ein- oder mehrfach herumverläuft, um quasi Umschlingungen des die Mikrowendel ausbildenden Drahtes zu erzeugen.

Grundsätzlich kann es sich bei der Mikrowendel, an der das Sicherungsmittel festgelegt ist, um die Okklusionswendel selbst oder auch um mit der Okklusionswendel verbundene Mikrowendeln handeln. Im letzteren Fall wird das Sicherungsmittel nur mittelbar mit der Okklusionswendel verbunden, was den Vorteil mit sich bringt, daß diese Ausführungsform besonders kostengünstig ist, weil zur Herstellung herkömmliche Okklusionswendeln verwendet werden können. Mit dem Sicherungsmittel verbundene Mikrowendeln können entsprechend über gängige Verfahren in die Okklusionswendel eingebracht werden. Zur Verbindung der Mikrowendel mit der Okklusionswendel eignen sich dabei dem Fachmann hinlänglich bekannte Verfahren wie Verschweißen, Verlöten, Verkleben oder mechanische Fügeverfahren. Typischerweise wird dabei sowohl am distalen als auch am proximalen Ende der Okklusionswendel eine kleinere Mikrowendel in diese eingebracht, wobei das Sicherungsmittel über die eingebrachten Mikrowendeln festgelegt ist.

Gemäß einer möglichen derartigen Ausführungsform der Erfindung wird in eine als Okklusionswendel dienende Mikrowendel mindestens eine weitere Mikrowendel eingelassen, deren Außendurchmesser dem Innendurchmesser der Okklusionswendel entspricht, wobei das Sicherungsmittel in zumindest einem Endbereich zwischen den Windungen der inneren Mikrowendel und den Windungen der Okklusionswendel eingeklemmt wird, um auf diese Weise eine reibschlüssige Verbindung herzustellen. Eine im proximalen Bereich eingebrachte innere Mikrowendel kann dabei gleichzeitig als Ablöseelement für die elektrolytische Ablösung der Okklusionswendel dienen. Die elektrolytische Ablösung von Okklusionswendeln ist dem zuständigen Fachmann hinlänglich bekannt und weist gegenüber anderen, im Stand der Technik bekannten Maßnahmen zur Ablösung von Okklusionswendeln vielfache Vorteile hinsichtlich Praktikabilität, Sicherung und Kostengünstigkeit auf. Hierbei werden in der Vorrichtung, zweckmäßigerweise in der Okklusionswendel eine oder mehrere voneinander beabstandete elektrolytisch korrodierbare Stellen vorgesehen, die in Verbindung mit einem elektrisch isolierenden Katheter und einer Spannungsquelle sowie einer Kathode, die üblicherweise auf der Körperoberfläche positioniert wird, die Ablösung durch elektrolytische Korrosion erlaubt. Die Okklusionswendel selbst dient dabei als Anode. Daneben sind aus dem Stand der Technik auch Vorrichtungen bekannt, bei denen die Ablösestelle im Führungsdraht vorgesehen ist.

Es ist dabei besonders zweckmäßig, wenn die Okklusionswendel, wie aus der DE 100 10 840 A1 bekannt, mehrere elektrolytisch korrodierbare Stellen aufweist, wobei in jedem zwischen diesen Stellen gelegenen Segment der Okklusionswendel ein Sicherungsmittel angeordnet ist, welches sich vorzugsweise jeweils von einem zum anderen Ende jedes Segmentes erstreckt. Diese Ausführungsform ermöglicht die Deponierung variabel dimensionierbarer Längen an Okklusionswendeln, wobei gleichzeitig durch die Sicherung jedes einzelnen, zwischen den elektrolytisch korrodierbaren Stellen angeordneten Segmentes, ein Höchstmaß an Sicherheit gegen das Abreißen der Okklusionswendel gewährleistet wird.

Neben der Möglichkeit, das Sicherungsmittel zwischen den Windungen der Okklusionswendel und den Windungen einer in der Okklusionswendel vorgesehenen Mikrowendel einzuklemmen, existiert die Alternative, in die Okklusionswendel eine Verdickung im Endbereich einzulassen, deren Außendurchmesser dem Innendurchmesser der Okklusionswendel entspricht, und das Sicherungsmittel zwischen den Windungen der Okklusionswendel und der Verdickung einzuklemmen. Für eine derartige Verdickung zur Festlegung des Sicherungsmittels über eine reibschlüssige Verbindung nach Art eines in die Okklusionswendel eingelassenen Stopfens sind verschiedenste Formen vorstellbar.

Um den erfindungsgemäßen "Rutschkupplungseffekt" hervorzurufen, reicht es aus, das Sicherungsmittel in einem seiner beiden Endbereiche durch reibschlüssige Verbindung in einer Mikrowendel festzulegen, während das Sicherungsmittel in seinem anderen Endbereich fest mit einer Mikrowendel verbunden ist. Da die Herstellung einer festen Verbindung zwischen Sicherungsmittel und Mikrowendel fertigungstechnisch einfacher ist, ist eine solche Lösung bevorzugt, wobei das Sicherungsmittel sowohl am proximalen als auch am distalen Ende fest mit einer Mikrowendel verbunden sein kann. Die Festlegung am distalen Ende gestaltet sich dabei aus fertigungstechnischer Sich problemloser. Selbstverständlich ist es jedoch auch möglich, das Sicherungsmittel in beiden Endbereichen durch reibschlüssige Verbindung in einer Mikrowendel festzulegen, so daß sich das Sicherungsmittel sowohl proximal als auch distal von der Mikrowendel lösen kann, wenn die auf die Vorrichtung ausgeübte Zugkraft einen bestimmten Wert überschreitet.

Zur Herstellung einer festen Verbindung zwischen Sicherungsmittel und Mikrowendel sind aus dem Stand der Technik übliche Verfahren einsetzbar, wie z. B. Verkleben, Aufschmelzen oder Anlöten, je nach Material, das für das Sicherungsmittel verwendet wird. Eine weitere Möglichkeit zur Festlegung des Sicherungsmittels am distalen Ende besteht darin, das Sicherungsmittel am distalen Ende an einer Verdickung zu befestigen, die sich distal der Mikrowendel befindet, wobei die Verdickung ein Durchgleiten durch die Mikrowendel verhindert, indem der Durchmesser der Verdickung größer als der Innendurchmesser der Mikrowendel gewählt wird. Die Verdickung kann beispielsweise die Form einer Kugel oder eines Balles haben. Auf diese Weise wird das Lösen des Sicherungsmittels vom distalen Ende verhindert, ohne eine unmittelbare feste Verbindung zwischen Mikrowendel und Sicherungsmittel erzeugt zu haben.

Darüber hinaus sind im Rahmen der Erfindung beliebige Kombinationen der möglichen reibschlüssigen und festen Verbindung am proximalen und distalen Ende möglich.

Gemäß einer besonders bevorzugten Ausführungsform handelt es sich bei dem Sicherungsmittel um einen Polymerfaden bzw. ein Polymerfadenbündel. Ein solcher Polymerfaden weist ausreichende Flexibilität auf, um ihn durch Windungslücken einer Mikrowendel hindurchzuführen oder um die Windungen einer Mikrowendel herumzuschlingen. Daneben kann ein solcher Polymerfaden auch nahezu beliebig dünn ausgebildet werden, so daß er als Sicherungsmittel für beliebige Okklusionswendeln einsetzbar ist, insbesondere auch für solche, die intrakraniell eingesetzt werden. Die Notwendigkeit, das Sicherungsmittel besonders dünn auszubilden, ergibt sich bereits daraus, daß die Lücken zwischen Windungen einer Mikrowendel lediglich in einer Größenordnung von 0,008 bis 0,01 mm liegen.

Als Polymere kommen vielfältige biokompatible Materialien in Frage, z. B. Polyester wie Dacron, Polyamide, insbesondere Nylon, Polyolefine, Polypropylene, Polybutylene etc. Darüber hinaus besteht auch die Möglichkeit, einzelne Metallfasern in den Polymerfaden einzuarbeiten, um auf diese Weise die Reißfestigkeit zu erhöhen. Auch wenn die Verwendung von Polymerfäden als Sicherungsmittel bevorzugt ist, so ist die Verwendung anderer Sicherungsmittel, insbesondere solcher auf Metallbasis, von dieser Erfindung keineswegs ausgeschlossen.

Als besonders vorteilhaft hat sich für die Herstellung der Polymerfäden die Verwendung von Polyamiden, insbesondere Nylon, herausgestellt. Durch die Verwendung von Polymerfäden als Sicherungsmittel kann ein zusätzlicher Effekt bewerkstelligt werden, wenn die Polymerfäden eine thrombogene Wirkung zeigen. Das Vorsehen von thrombogenen Fäden an Okklusionsmitteln ist grundsätzlich aus dem Stand der Technik bekannt, beispielsweise aus der europäischen Patentanmeldung EP 0 800 791 A1 oder dem US-Patent 5382259. Fasern mit einer thrombogenen Wirkung fördern im zu verschließenden Körperhohlraum, insbesondere einem Aneurysma, die Thrombusbildung und gewährleisten auf diese Weise das effektive Verschließen des Aneurysmas. Eine weitere Verbesserung kann durch Beschichtung des Polymerfadens bzw. des Sicherungsmittels und/oder der Okklusionswendel mit Kollagen erzielt werden.

Damit der Polymerfaden die gewünschte Wirkung erzielen kann, ist es zweckmäßig, ihn an einer oder mehreren Stellen nach außen aus der Okklusionswendel herausragen zu lassen. Insbesondere können die Enden des Polymerfadens aus der Okklusionswendel herausragen, wenn die Polymerfäden im Endbereich zwischen den Windungen der Okklusionswendel eingeklemmt werden. Wenn der Polymerfaden mehrfach zwischen den Windungen hin- und herläuft, ergeben sich dementsprechend mehrere Stellen, an denen der Polymerfaden aus der Mikrowendel heraussteht, was die thrombogene Wirkung erhöht.

Neben dem Vorsehen von Positionen im Endbereich der Okklusionswendel, an denen der Polymerfaden aus dieser nach außen heraussteht, ist es auch möglich, den Polymerfaden zwischen proximalem und distalem Ende in Form von Schlaufen an einer oder mehreren Stellen nach außen aus der Okklusionswendel herausstehen zu lassen. Eine solche Schlaufe kann durch die Lücke zwischen zwei Windungen hindurchgeführt werden oder auch eine oder mehrere Windungen umschließen. Entsprechend ist es grundsätzlich möglich, entlang der gesamten Länge der Okklusionswendel und des Polymerfadens diesen teilweise nach außen stehen zu lassen, um auf diese Weise die thrombogene Wirkung zur Entfaltung kommen zu lassen. Um zu verhindern, daß sich eine solche Schlaufe in das Lumen der Mikrowendel zurückzieht, sobald eine Zugkraft auf sie ausgeübt wird, ist es zweckmäßig, den Polymerfaden neben den Schlaufen um einzelne Windungen der Okklusionswendel zusätzlich herumzuschlingen, um auf diese Weise die Position der Schlaufe festzulegen. Entsprechend kann der Polymerfaden sowohl den erfindungsgemäßen Zweck als Sicherungsmittel erfüllen, als auch thrombogene Wirkung entfalten.

Eine weitere Möglichkeit, die erfindungsgemäße Wirkung mit der thrombogenen Wirkung von Polymerfäden zu verknüpfen, besteht darin, um das längs durch das Lumen der Okklusionswendel verlaufende Sicherungsmittel einzelne, kürzere Polymerfäden zu schlingen, deren Enden nach außen aus der Okklusionswendel herausstehen. Das Sicherungsmittel kann auch zusammen mit den kürzeren Polymerfäden erwärmt werden, so daß das Sicherungsmittel und/oder die kürzeren Polymerfäden aufweichen und eine Verklebung stattfindet. Es handelt sich somit um ein Aufschmelzen der Polymerfäden auf das innere Sicherungsmittel. Eine weitere Möglichkeit, die kürzeren Polymerfäden mit dem innen verlaufenden Sicherungsmittel zu verbinden, besteht darin, das innere Sicherungsmittel mit einer Klebebeschichtung zu versehen, die die Polymerfäden festhält. Schließlich sind die beiden letztgenannten Verfahren auch kombinierbar. Besonders vorteilhaft ist es, die kürzeren Polymerfäden zunächst um das Sicherungsmittel herumzuschlingen und anschließend zur Erzielung einer Verklebung zu erwärmen und/oder die Polymerfäden um ein mit Klebebeschichtung versehenes Sicherungsmittel herumzuschlingen.

Die Positionen der Polymerfäden werden darüber hinaus dadurch fixiert, daß sie im Endbereich durch die Okklusionswendel verlaufen und entsprechend zwischen den Windungen eingeklemmt sind. Die Fixierung von Polymerfäden, die eine thrombogene Wirkung entfalten können, ist besonders wichtig, weil losgelöste Polymerfäden eine Thrombusbildung an unerwünschter Stelle verursachen können und zudem schwer auffindbar sind. Naturgemäß ist mit der Thrombusbildung in wichtigen Blutgefäßen eine erhebliche Gefahr für den Patienten verbunden.

Typischerweise besteht ein Polymerfaden aus einzelnen, miteinander verdrillten oder versponnenen Fasern. Zur Erzielung der thrombogenen Wirkung reicht es daher aus, wenn lediglich einige Fasern eines Polymerfadens nach außen aus der Okklusionswendel herausstehen, während andere Fasern praktisch entlang ihrer gesamten Länge durch das Lumen der Okklusionswendel verlaufen, um den erfindunsgemäßen Effekt als Sicherungsmittel zu erzielen. Es können auch gezielt einzelne Fasern in den Polymerfaden eingebaut werden, die kürzer sind als der Polymerfaden selbst und deren Enden nach außen aus der Okklusionswendel herausstehen. Bei einer ausreichenden Anzahl einzelner Fasern kann das Okklusionsmittel praktisch entlang seiner gesamten Länge mit nach außen stehenden thrombogenen Fasern versehen werden. Die Fixierung der thrombogenen Fasern wird dabei wiederum durch Hindurchführen der Fasern zwischen den Windungen der Okklusionswendel gewährleistet.

Einzelne, nach außen aus der Okklusionswendel herausstehende Fasern können auch, anstatt mit dem als Sicherungsmittel dienenden Polymerfaden versponnen zu sein, auf dem Sicherungsmittel aufgeklebt oder aufgeschmolzen werden. Dies ist grundsätzlich auch dann möglich, wenn es sich bei dem Sicherungsmittel nicht um einen Polymerfaden sondern beispielsweise um einen Metallfaden handelt. Um Fasern aufschmelzen zu können, ist es dabei sinnvoll, Thermoplaste wie Polyamide als Fasermaterial zu verwenden.

Vorzugsweise ist das Sicherungsmittel der erfindungsgemäßen Vorrichtung etwas länger dimensioniert als der Teilbereich der Mikrowendel, über den es sich erstreckt. Diese Längendimensionierung des Sicherungsmittels gewährleistet eine weniger rigide Anordnung trotz Festlegung in der Mikrowendel, so daß das Sicherungsmittel in der Mikrowendel ohne äußere Einwirkung keiner Zugbeanspruchung unterliegt und die Flexibilität der Mikrowendel nicht eingeschränkt wird. Das Sicherungsmittel kann sich auch über die gesamte Länge der Okklusionswendel vom proximalen bis zum distalen Ende erstrecken, ohne daß Einbußen an Beweglichkeit und Flexibilität zu verzeichnen sind, um auf diese Weise die gesamte Okklusionswendel gegen ein Abreißen zu sichern. Da die distale Spitze einer Okklusionswendel bei der Einbringung in ein Blutgefäß einer besonders hohen Beanspruchung unterliegt, sollte sich das Sicherungsmittel insbesondere bis zum distalen Spitzenabschnitt der Okklusionswendel erstrecken.

Zur Herstellung von Mikrowendeln und Okklusionswendeln haben sich aufgrund ihrer geringen Traumatisierung Platin und Platinlegierungen, insbesondere Platin-Iridium-Legierungen bewährt. Die Okklusionswendel kann auch zu einer übergeordneten Struktur vorgeformt sein, die sie erst nach Ausbringung aus dem Katheter im Aneurysma ausbildet. Auf diese Weise wird das Aneurysma besonders effektiv ausgefüllt. Vorteilhafterweise schließt sich proximal an die Okklusionswendel eine als Führungsdraht ausgebildete Einführhilfe an.

Neben einer Vorrichtung zur Implantation von Mikrowendeln in Körperhohlräumen und Blutgefäßen betrifft die Erfindung auch die Okklusionswendel zur Verwendung in Verbindung mit der erfindungsgemäßen Vorrichtung selbst.

Die Erfindung wird anhand der in den Zeichnungen veranschaulichten Ausführungsbeispiele näher erläutert. Es zeigen:
- Figur 1: den Längsschnitt durch eine erfindungsgemäße Vorrichtung (ohne Katheter) in Seitenansicht im proximalen und distalen Bereich gemäß einer ersten Ausführungsform der Erfindung;
- Figur 2: den Längsschnitt durch eine erfindungsgemäße Vorrichtung (ohne Katheter) in Seitenansicht im proximalen und distalen Bereich gemäß einer zweiten Ausführungsform der Erfindung;
- Figur 3: den Längsschnitt durch eine erfindungsgemäße Vorrichtung (ohne Katheter, distale Spitze und Ablöseelement) in Seitenansicht im proximalen und distalen Bereich gemäß einer dritten Ausführungsform der Erfindung und
- Figur 4: den Längsschnitt durch eine erfindungsgemäße Vorrichtung (ohne Katheter, distale Spitze und Ablöseelement) in Seitenansicht im proximalen und distalen Bereich gemäß einer vierten Ausführungsform der Erfindung.

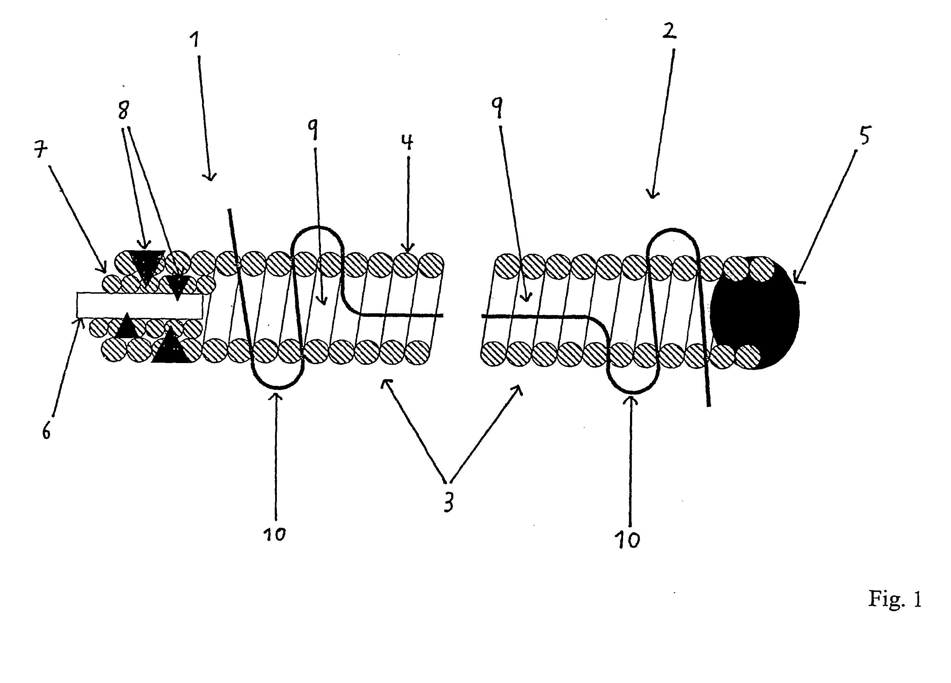

In Figur 1 sind der proximale Bereich 1 und der distale Bereich 2 einer Okklusionswendel 3 im Längsschnitt dargestellt. Die Okklusionswendel 3 wird dabei durch einen aus einer Vielzahl von Windungen 4 bestehenden Draht gebildet. Die distale Spitze 5 der Okklusionswendel 3 ist dabei abgerundet, um die Gefahr einer Traumatisierung eines Aneurysmas zu minimieren. Proximal schließt sich an die Okklusionswendel 3 ein Ablöseelement 6 an, das durch eine zusätzliche in die Okklusionswendel 3 eingebrachte Mikrowendel 7 verläuft. Die Verbindung zwischen zusätzlicher Mikrowendel 7 und Okklusionswendel 3 bzw. Ablöseelement 6 und zusätzlicher Mikrowendel 7 ist durch Fügestellen 8 hergestellt, wobei hier verschiedene Techniken wie Löten, Schweißen, Kleben oder mechanische Fügeverfahren zum Einsatz kommen können. Das Ablöseelement 6 ist elektrolytisch korrodierbar ausgebildet, um durch Anlegen einer Spannung die Okklusionswendel 3 freizusetzen und im Aneurysma zu plazieren.

Durch das Lumen 9 der Okklusionswendel 3 verläuft in Längsrichtung ein als Sicherungsmittel 10 dienender Polymerfaden, der sowohl im proximalen als auch im distalen Endbereich mehrfach so zwischen den Windungen 4 der Okklusionswendel 3 hin- und herverläuft, daß er auf diese Weise durch eine reibschlüssige Verbindung in der Okklusionswendel 3 festgelegt ist. Bei Überschreiten einer bestimmten Zugkraft kann jedoch der Polymerfaden 10 aus den Windungen 4 herausrutschen. Die Kraft, die überschritten werden muß, um ein Herausrutschen zu bewirken, kann durch die Anzahl der Verläufe des Polymerfadens 10 zwischen einzelnen Windungen 4 der Okklusionswendel 3 eingestellt werden. Naturgemäß nimmt die maximale Zugkraft zu, wenn der Polymerfaden 10 häufiger durch die Windungen 4 hindurchgeführt wird. In seinen Endbereichen steht der Polymerfaden 10 zudem mehrfach aus der Okklusionswendel 3 heraus und kann daher eine thrombogene Wirkung entfalten.

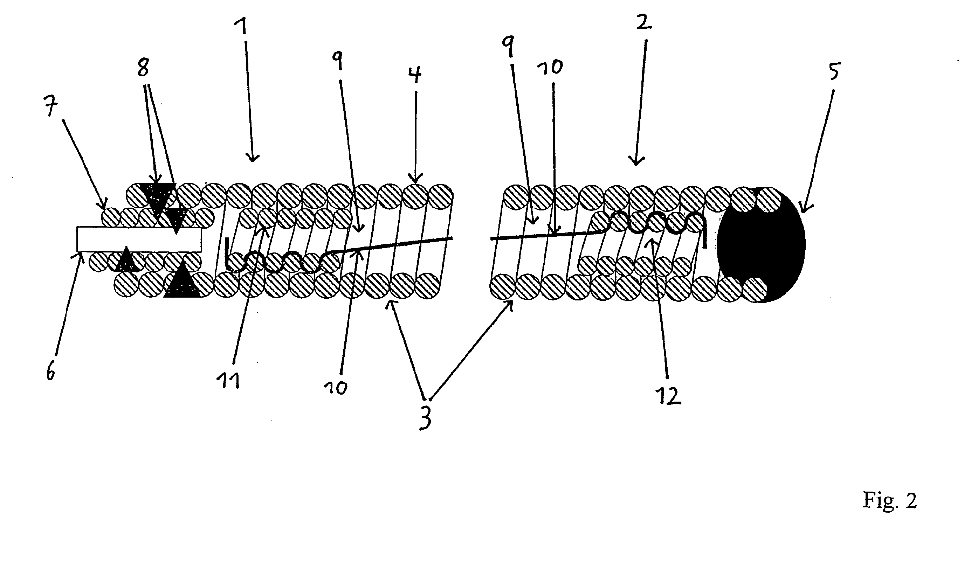

In Figur 2 ist eine alternative Ausführungsform der Erfindung gezeigt, bei der sowohl im proximalen Bereich 1 als auch im distalen Bereich 2 der Okklusionswendel 3 eine zusätzliche Mikrowendel 11, 12 eingebracht ist, deren Außendurchmesser dem Innendurchmesser der Okklusionswendel 3 entspricht. Die inneren Mikrowendeln 11, 12 können in die Okklusionswendel 3 eingeschraubt und durch Laserschweißtechnik, Verlöten oder Verkleben mit der Okklusionswendel 3 verbunden werden. Der als Sicherungsmittel 10 dienende Polymerfaden wird dabei sowohl im distalen als auch im proximalen Bereich zwischen den Windungen der inneren Mikrowendel 11, 12 und den Windungen 4 der Okklusionswendel 3 eingeklemmt, um ihn auf diese Weise reibschlüssig festzulegen. In diesem Fall steht der Polymerfaden 10 nicht nach außen aus der Okklusionswendel 3 heraus, so daß eine zusätzliche thrombogene Wirkung nicht erzielt wird. Selbstverständlich ist jedoch auch bei Verwendung zusätzlicher Mikrowendeln 11, 12 das Herausstehenlassen des Polymerfadens 10 aus der Okklusionswendel 3 möglich. Des weiteren ist es möglich, die innere Mikrowendel 11, 12 so auszubilden, daß sie sowohl mit dem Polymerfaden 10 als auch mit dem Ablöseelement 6 verbunden ist, indem die inneren Mikrowendeln 7 und 11 bzw. 7 und 12 kombiniert werden.

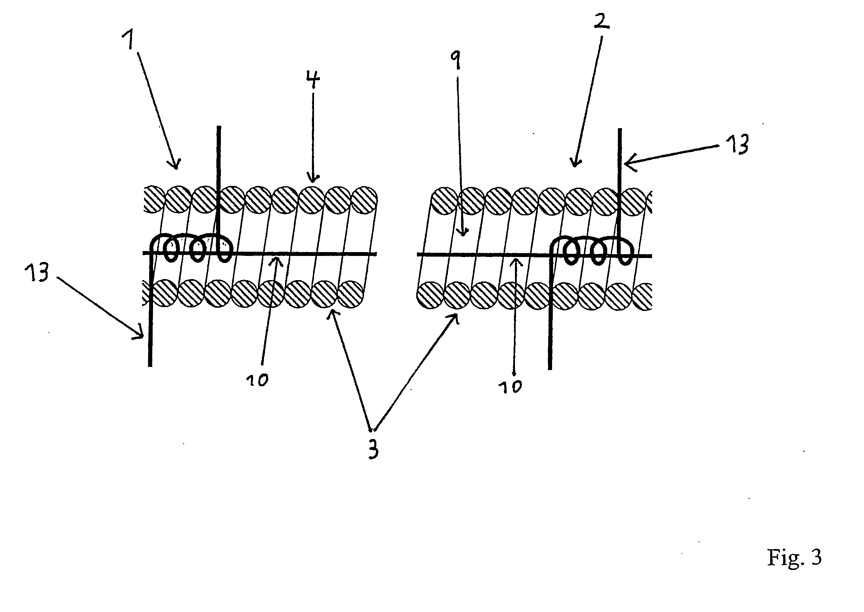

Gemäß einer dritten Ausführungsform der Erfindung, die in Figur 3 gezeigt ist, verläuft das Sicherungsmittel 10 sowohl im proximalen Bereich 1 als auch im distalen Bereich 2 der Okklusionswendel 3 durch das Lumen 9. Das Sicherungsmittel 10 ist im proximalen und/oder distalen Endbereich reibschlüssig in der Okklusionswendel 3 festgelegt, um auf diese Weise den erfindungsgemäßen Effekt erzielen zu können. Darüber hinaus sind um das Sicherungsmittel 10 einzelne, kürzere Polymerfäden 13 geschlungen, deren Enden nach außen aus der Okklusionswendel 3 herausstehen. Die herausstehenden Polymerfäden 13 dienen der Erzielung einer thrombogenen Wirkung in den zu verschließenden Körperhohlräumen, insbesondere Aneurysmen. Im Endbereich verlaufen die Polymerfäden 13 durch die Windungen 4 der Okklusionswendel 3, so daß sie zwischen den Windungen 4 eingeklemmt und auf diese Weise fixiert sind.

Vorteilhafterweise werden die Polymerfäden 13 zusätzlich dadurch mit dem Sicherungsmittel 10 verbunden, daß sie zusammen mit dem Sicherungsmittel 10 erwärmt werden, so daß die Polymerfäden 13 und/oder das Sicherungsmittel 10, bei dem es sich ebenfalls um einen Polymerfaden handeln kann, aufweichen und eine Verklebung stattfindet. Eine weitere Möglichkeit, die Polymerfäden 13 am Sicherungsmittel 10 zu fixieren, besteht darin, das Sicherungsmittel 10 mit einer Klebebeschichtung zu versehen. Der besseren Darstellbarkeit halber sind die Polymerfäden 13 in Figur 3 lediglich um das Sicherungsmittel 10 herumgeschlungen dargestellt, nicht jedoch fest fixiert.

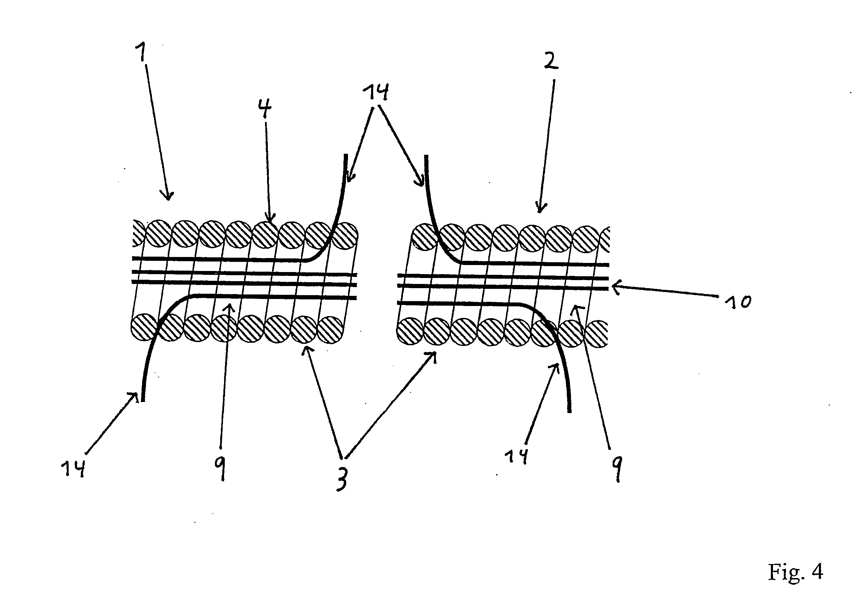

Auch gemäß der weiteren Ausführungsform, die in Figur 4 gezeigt ist, verläuft das Sicherungsmittel 10 durch das Lumen 9 der Okklusionswendel 3 zwischen dem proximalen Bereich 1 und dem distalen Bereich 2. Zur Erzielung des erfindungsgemäßen Effekts ist das Sicherungsmittel 10 in mindestens einem Endbereich reibschlüssig mit der Okklusionswendel 3 verbunden. In diesem Fall handelt es sich bei dem Sicherungsmittel 10 um einen Polymerfaden, der aus einzelnen Fasern besteht. Dabei verlaufen einige Faser praktisch entlang ihrer gesamten Länge durch das Lumen 9 der Okklusionswendel 3, während andere Fasern 14 kürzer sind als das Sicherungsmittel 10 insgesamt und mit ihren Enden nach außen aus der Okklusionswendel 3 herausstehen. Die herausstehenden Fasern 14 dienen wiederum der Erzielung einer thrombogenen Wirkung. Eine Fixierung der thrombogenen Fasern 14 findet durch Hindurchführen durch die Windungen 4 der Okklusionswendel 3 statt, so daß die Fasern 14 in ihrem Endbereich quasi zwischen den Windungen 4 eingeklemmt sind. Zusätzlich ist es sinnvoll die thrombogenen Fasern 14 in das Sicherungsmittel 10 einzubauen, in dem sie mit dem das Sicherungsmittel 10 bildenden Polymerfaden versponnen oder verdrillt werden. Alternativ können die Fasern 14 auch auf dem Sicherungsmittel 10 aufgeklebt oder aufgeschmolzen werden.

Um das Prinzip der Erfindung übersichtlicher darzustellen, ist die Verdrillung der Fäden 14 mit dem Sicherungsmittel 10 in Figur 4 nicht gezeigt.

## Patentansprüche

1. Vorrichtung zur Implantation von Mikrowendeln (3, 7, 11, 12) in Körperhohlräumen und Blutgefäßen, insbesondere Aneurysmen, wobei die Mikrowendeln (3, 7, 11, 12) aus einer Vielzahl von Windungen (4) bestehende Drähte sind, mindestens eine Mikrowendel (3, 7,11,12) als Okklusionswendel (3) dem Verschließen des Körperhohlraumes oder Blutgefäßes dient und die Vorrichtung aus einem Katheter, einer oder mehreren im Katheter in Längsrichtung verschiebbaren Mikrowendeln (3, 7, 11, 12) und mindestens einem das Lumen (9) der Okklusionswendel (3) zumindest teilweise durchlaufenden Sicherungsmittel (10) besteht und das Sicherungsmittel (10) in seinen Endbereichen in der Mikrowendel (3, 7, 11, 12) festgelegt ist, **dadurch gekennzeichnet, daß** das Sicherungsmittel (10) zumindest in einem Endbereich zur Herstellung einer reibschlüssigen Verbindung so in einer Mikrowendel (3, 7, 11, 12) festgelegt ist, daß diese Verbindung durch Überschreiten einer bestimmten auf das Sicherungsmittel (10) wirkenden Zugkraft von der Mikrowendel (3, 7, 11, 12) lösbar ist.

2. Vorrichtung nach Anspruch 1, **dadurch gekennzeichnet, daß** das Sicherungsmittel (10) in zumindest einem Endbereich zwischen einzelnen oder mehreren Windungen (4) einer Mikrowendel (3, 7,11,12) eingeklemmt ist.

3. Vorrichtung nach Anspruch 1 oder 2, **dadurch gekennzeichnet, daß** das Sicherungsmittel (10) in zumindest einem Endbereich um den die Windungen (4) bildenden Draht einer Mikrowendel (3, 7,11,12) ein- oder mehrfach herumverläuft.

4. Vorrichtung nach einem der Ansprüche 1 bis 3, **dadurch gekennzeichnet, daß** in eine als Okklusionswendel (3) dienende Mikrowendel mindestens eine weitere Mikrowendel (11,12) eingelassen ist, deren Außendurchmesser dem Innendurchmesser der Okklusionswendel (3) entspricht, und das Sicherungsmittel (10) in zumindest einem Endbereich zwischen den Windungen der inneren Mikrowendel (11, 12) und den Windungen (4) der Okklusionswendel (3) eingeklemmt ist.

5. Vorrichtung nach einem der Ansprüche 1 bis 4, **dadurch gekennzeichnet, daß** in einer als Okklusionswendel (3) dienenden Mikrowendel eine Verdickung eingelassen ist, deren Außendurchmesser dem Innendurchmesser der Okklusionswendel (3) entspricht, und das Sicherungsmittel (10) in zumindest einem Endbereich zwischen den Windungen (4) der Okklusionswendel (3) und der Verdickung eingeklemmt ist.

6. Vorrichtung nach einem der Ansprüche 1 bis 5, **dadurch gekennzeichnet, daß** das Sicherungsmittel (10) in beiden Endbereichen durch reibschlüssige Verbindung in einer Mikrowendel (3, 7,11,12) festgelegt ist.

7. Vorrichtung nach einem der Ansprüche 1 bis 5, **dadurch gekennzeichnet, daß** das Sicherungsmittel (10) im distalen Endbereich fest und im proximalen Endbereich reibschlüssig mit einer Mikrowendel (3, 7,11,12) verbunden ist.

8. Vorrichtung nach einem der Ansprüche 1 bis 5, **dadurch gekennzeichnet, daß** das Sicherungsmittel (10) im proximalen Endbereich fest und im distalen Endbereich reibschlüssig mit einer Mikrowendel (3, 7, 11, 12) verbunden ist.

9. Vorrichtung nach Anspruch 8, **dadurch gekennzeichnet, daß** das Sicherungsmittel (10) am distalen Ende an einer sich distal einer Mikrowendel befindenden Verdickung befestigt ist, die ein Durchgleiten durch die Mikrowendel verhindert.

10. Vorrichtung nach einem der Ansprüche 1 bis 9, **dadurch gekennzeichnet, daß** das Sicherungsmittel (10) ein Polymerfaden oder Polymerfadenbündel ist.

11. Vorrichtung nach Anspruch 10, **dadurch gekennzeichnet, daß** der Polymerfaden (10) eine thrombogene Wirkung hat.

12. Vorrichtung nach Anspruch 11, **dadurch gekennzeichnet, daß** der Polymerfaden (10) aus einem Polyamid besteht.

13. Vorrichtung nach einem der Ansprüche 10 bis 12, **dadurch gekennzeichnet, daß** das Sicherungsmittel (10) und/oder die Okklusionswendel (3) kollagenbeschichtet sind.

14. Vorrichtung nach einem der Ansprüche 10 bis 13, **dadurch gekennzeichnet, daß** der Polymerfaden (10) an einer oder mehreren Stellen nach außen aus der Okklusionswendel (3) heraussteht.

15. Vorrichtung nach Anspruch 14, **dadurch gekennzeichnet, daß** der Polymerfaden (10) an einer oder mehreren Stellen zwischen proximalem und distalem Ende der Okklusionswendel (3) in Form von Schlaufen nach außen aus der Okklusionswendel (3) heraussteht.

16. Vorrichtung nach Anspruch 15, **dadurch gekennzeichnet, daß** der Polymerfaden (10) neben den Schlaufen um einzelne Windungen (4) der Okklusionswendel (3) herumgeschlungen ist.

17. Vorrichtung nach einem der Ansprüche 1 bis 16, **dadurch gekennzeichnet, daß** um das Sicherungsmittel (10) einzelne kürzere Polymerfäden (13) geschlungen sind, deren Enden nach außen aus der Okklusionswendel (3) herausstehen.

18. Vorrichtung nach einem der Ansprüche 1 bis 17, **dadurch gekennzeichnet, daß** das Sicherungsmittel (10) mit einer Klebebeschichtung versehen ist, die einzelne kürzere Polymerfäden (13) mit dem Sicherungsmittel (10) verbindet, deren Enden nach außen aus der Okklusionswendel 3) herausstehen.

19. Vorrichtung nach einem der Ansprüche 1 bis 18, **dadurch gekennzeichnet, daß** auf das Sicherungsmittel (10) einzelne kürzere Polymerfäden (13) aufgeschmolzen sind, deren Enden nach außen aus der Okklusionswendel (3) herausstehen.

20. Vorrichtung nach einem Ansprüche 10 bis 19, **dadurch gekennzeichnet, daß** der Polymerfaden (10) aus einzelnen Fasern besteht.

21. Vorrichtung nach Anspruch 20, **dadurch gekennzeichnet, daß** einige Fasern (14) des Polymerfadens (10) nach außen aus der Okklusionswendel (3) herausstehen, während andere Fasern des Polymerfadens (10) das Lumen (9) der Okklusionswendel (3) entlang ihrer gesamten Länge durchlaufen.

22. Vorrichtung nach Anspruch 20 oder 21, **dadurch gekennzeichnet, daß** einzelne Fasern (14) des Polymerfadens (10) kürzer sind als der Polymerfaden (10) im ganzen und ihre Enden nach außen aus der Okklusionswendel (3) herausstehen.

23. Vorrichtung nach einem der Ansprüche 1 bis 22, **dadurch gekennzeichnet, daß** einzelne Fasern auf dem Sicherungsmittel (10) aufgeklebt oder aufgeschmolzen sind und nach außen aus der Okklusionswendel (3) herausstehen.

24. Vorrichtung nach einem der Ansprüche 1 bis 23, **dadurch gekennzeichnet, daß** die Okklusionswendel (3) mehrere elektrolytisch korrodierbare Stellen (6) aufweist, wobei in jedem zwischen diesen Stellen (6) gelegenen Segment der Okklusionswendel (3) ein Sicherungsmittel (10) angeordnet ist.

25. Okklusionswendel zur Verwendung in Verbindung mit einer Vorrichtung nach einem der Ansprüche 1 bis 24.

## Claims

1. Device for the implantation of microcoils (3, 7, 11, 12) into body cavities and blood vessels, in particular aneurysms, with said microcoils (3, 7, 11,12) comprising wires forming a plurality of windings (4), at least one microcoil (3, 7, 11, 12) serving as occlusion helix (3) for the occlusion of the body cavity or blood vessel, and the device consisting of a catheter, one or several microcoils (3, 7, 11, 12) movably arranged within the catheter in longitudinal direction and at least one securing means (10) passing at least partially through the lumen (9) of the occlusion helix (3), with said securing means (10) being fixed in its end areas inside the microcoil (3, 7, 11, 12), **characterized in that,** the securing means (10) in order to achieve a frictional connection is fixed in the a microcoil (3, 7, 11, 12) at least in one end area in such a manner that this connection is detachable from the microcoil (3, 7, 11, 12) when a certain tensile force acting on the securing means (10) is exceeded.

2. Device according to claim 1, **characterized in that** the securing means (10) is clamped in at least one end area between individual or several windings (4) of a microcoil (3,7,11,12).

3. Device according to claim 1 or 2, **characterized in that** the securing means (10) passes in at least one end area in one or several turns around the wire forming the windings (4) of a microcoil (3, 7, 11, 12).

4. Device according to at least one of the claims 1 to 3, **characterized in that** at least one further microcoil (11, 12) is placed in a microcoil serving as occlusion helix (3), with the outside diameter of said former microcoil corresponding to the inside diameter of the occlusion helix (3), and the securing means (10) is clamped in at least one end area between the windings of the inner microcoil (11,12) and the windings (4) of the occlusion helix (3).

5. Device according to any one of the claims 1 to 4, **characterized in that** a thickening element is placed in a microcoil serving as occlusion helix (3), the outside diameter of said thickening element corresponding to the inside diameter of the occlusion helix (3), and the securing means (10) is clamped in at least one end area between the windings (4) of the occlusion helix (3) and the thickening element.

6. Device according to any one of the claims 1 to 5, **characterized in that** the securing means (10) is fixed in both end areas in a microcoil (3, 7, 11, 12) by means of a frictional connection.

7. Device according to any one of the claims 1 to 5, **characterized in that** the securing means (10) is frictionally connected in the distal end area and in the proximal end area to a microcoil (3, 7, 11, 12).

8. Device according to any one of the claims 1 to 5, **characterized in that** the securing means (10) is permanently connected in the proximal end area and frictionally connected in the distal end area to a microcoil (3, 7, 11, 12).

9. Device according to claim 8, **characterized in that** the securing means (10) is attached at the distal end to a thickening element arranged distally of a microcoil with said element preventing passage through the microcoil.

10. The device according to any one of the claims 1 to 9, **characterized in that** the securing means (10) is a polymer thread or polymer thread bundle.

11. The device according to claim 10, **characterized in that** the polymer thread (10) has a thrombogeneous effect.

12. The device according to claim 11, **characterized in that** the polymer thread (10) consists of a polyamide.

13. The device according to any one of the claims 10 to 12, **characterized in that** the securing means (10) and/or the occlusion helix (3) is provided with a collagen coating.

14. The device according to any one of claims 10 to 13, **characterized in that** the polymer thread (10) projects outwardly from the occlusion helix (3) at one or several locations.

15. Device according to claim 14, **characterized in that** the polymer thread (10) projects outwardly from and produces loops on the occlusion helix (3) at one or several locations between the proximal and distal end of the occlusion helix (3).

16. Device according to claim 15, **characterized in that** adjacent to the loops the polymer thread (10) is wrapped around individual windings (4) of the occlusion helix (3).

17. The device according to any one of claims 1 to 16, **characterized in that** individual, shorter polymer threads (13) are wrapped around the securing means (10) with the ends of said threads projecting outwardly from the occlusion helix (3).

18. The device according to any one of claims 1 to 17, **characterized in that** the securing means (10) is provided with an adhesive coating causing individual, shorter polymer threads (13) to be bonded to the securing means (10) with the ends of said threads projecting outwardly from the occlusion helix (3).

19. The device according to any one of claims 1 to 18, **characterized in that** individual, shorter polymer threads (13) are fused onto the securing means (10) with the ends of said threads projecting outwardly from the occlusion helix (3).

20. The device according to any one of claims 10 to 19, **characterized in that** the polymer thread (10) consists of individual fibers.

21. Device according to claim 20, **characterized in that** some fibers (14) of the polymer thread (10) project outwardly from the occlusion helix (3) whereas other fibers of the polymer thread (10) pass through the lumen (9) of the occlusion helix (3) along its entire length.

22. Device according to claim 20 or 21, **characterized in that** individual fibers (14) of the polymer thread (10) are shorter than the polymer thread (10) in its entirety and that their ends project outwardly from the occlusion helix (3).

23. The device according to any one of claims 1 to 22, **characterized in that** individual fibers are glued to or fused onto the securing means (10) and project outwardly from the occlusion helix (3).

24. The device according to any one of the claims 1 to 23, **characterized in that** the occlusion helix (3) has several locations (6) that are electrolytically corrodible, and that in each segment of the occlusion helix (3) a securing means (10) is arranged between these locations (6)

25. Occlusion helix for use in connection with a device according to any one of the claims 1 to 24.

## Revendications

1. Dispositif d'implantation de micro-hélices **(3, 7, 11,** 12) dans des cavités corporelles et des vaisseaux sanguins, en particulier des anévrismes, dans lequel les micro-hélices sont des fils constitués d'une pluralité d'enroulements **(4),** au moins une micro-hélice **(3, 7, 11, 12)** sert, en tant qu'hélice d'occlusion **(3),** à fermer la cavité corporelle ou le vaisseau sanguin et le dispositif est constitué d'un cathéter, d'une ou plusieurs micro-hélices **(3, 7, 11, 12)** mobiles dans la direction longitudinale dans le cathéter et d'au moins un moyen d'immobilisation **(10)** traversant au moins partiellement l'ouverture **(9)** de l'hélice d'occlusion **(3),** et le moyen d'immobilisation **(10)** est fixé dans ses zones d'extrémité dans la micro-hélice **(3, 7, 11, 12), caractérisé en ce qu'**afin d'établir une liaison par frottement, le moyen d'immobilisation (10) est fixé dans une micro-hélice **(3, 7, 11, 12)** au moins dans une zone d'extrémité, de telle manière que cette liaison peut être levée sur la micro-hélice **(3, 7, 11, 12)** par dépassement d'une force de traction déterminée agissant sur le moyen d'immobilisation **(10).**

2. Dispositif selon la revendication 1, **caractérisé en ce que** le moyen d'immobilisation **(10)** est bloqué, dans au moins une zone d'extrémité, entre un ou plusieurs enroulements **(4)** d'une micro-hélice **(3, 7, 11, 12).**

3. Dispositif selon la revendication 1 ou 2, **caractérisé en ce que** le moyen d'immobilisation **(10)** tourne une ou plusieurs fois, dans au moins une zone d'extrémité, autour d'un fil d'une micro-hélice **(3, 7, 11, 12)** formant les enroulements **(4).**

4. Dispositif selon l'une des revendications 1 à 3, **caractérisé en ce que** dans une micro-hélice servant d'hélice d'occlusion **(3)** est insérée une autre micro-hélice **(11, 12)** dont le diamètre extérieur correspond au diamètre intérieur de l'hélice d'occlusion **(3),** et le moyen d'immobilisation **(10)** est coincé dans au moins une zone d'extrémité entre les enroulements de la micro-hélice intérieure **(11, 12)** et les enroulements **(4)** de l'hélice d'occlusion **(3).**

5. Dispositif selon l'une des revendications 1 à 4, **caractérisé en ce que** dans une micro-hélice servant d'hélice d'occlusion **(3)** est inséré un épaississement dont le diamètre extérieur correspond au diamètre intérieur de l'hélice d'occlusion **(3),** et le moyen d'immobilisation **(10)** est coincé dans au moins une zone d'extrémité entre les enroulements **(4)** de l'hélice d'occlusion **(3)** et l'épaississement.

6. Dispositif selon l'une des revendications 1 à 5, **caractérisé en ce que** le moyen d'immobilisation **(10)** est fixé dans deux zones d'extrémité par liaison par frottement dans une micro-hélice **(3, 7, 11, 12).**

7. Dispositif selon l'une des revendications 1 à 5, **caractérisé en ce que** le moyen d'immobilisation **(10)** est relié à une micro-hélice **(3, 7, 11, 12)** de manière fixe dans la zone d'extrémité distale et par frottement dans la zone d'extrémité proximale.

8. Dispositif selon l'une des revendications 1 à 5, **caractérisé en ce que** le moyen d'immobilisation **(10)** est relié à une micro-hélice **(3, 7, 11, 12)** de manière fixe dans la zone d'extrémité proximale et par frottement dans la zone d'extrémité distale.

9. Dispositif selon la revendication 8, **caractérisé en ce que** le moyen d'immobilisation **(10)** est fixé à l'extrémité distale à un épaississement situé en position distale par rapport à une micro-hélice, qui empêche un glissement à travers la micro-hélice.

10. Dispositif selon l'une des revendications 1 à 9, **caractérisé en ce que** le moyen d'immobilisation **(10)** est un fil polymère ou un faisceau de fils polymères.

11. Dispositif selon la revendication 10, **caractérisé en ce que** le fil polymère **(10)** a un effet thrombogène.

12. Dispositif selon la revendication 11, **caractérisé en ce que** le fil polymère **(10)** est constitué de polyamide.

13. Dispositif selon l'une des revendications 10 à 12, **caractérisé en ce que** le moyen d'immobilisation **(10)** et/ou l'hélice d'occlusion **(3)** est/sont recouvert(s) de collagène.

14. Dispositif selon l'une des revendications 10 à 13, **caractérisé en ce que** le fil polymère **(10)** sort de l'hélice d'occlusion (3) vers l'extérieur en un ou plusieurs endroits.

15. Dispositif selon la revendication 14, **caractérisé en ce que** le fil polymère **(10)** sort de l'hélice d'occlusion **(3)** vers l'extérieur sous la forme de boucles en un ou plusieurs endroits entre l'extrémité proximale et l'extrémité distale de l'hélice d'occlusion **(3).**

16. Dispositif selon la revendication 15, **caractérisé en ce que** le fil polymère **(10)** est enroulé autour de certains enroulements **(4)** de l'hélice d'occlusion **(3)** à côté des boucles.

17. Dispositif selon l'une des revendications 1 à 16, **caractérisé en ce qu'**autour du moyen d'immobilisation **(10)** sont enroulés différents fils polymères **(13)** plus courts, dont les extrémités sortent vers l'extérieur depuis l'hélice d'occlusion **(3).**

18. Dispositif selon l'une des revendications 1 à 17, **caractérisé en ce que** le moyen d'immobilisation **(10)** est muni d'un revêtement adhésif qui relie différents fils polymères **(13)** plus courts avec le moyen d'immobilisation **(10),** dont les extrémités sortent vers l'extérieur depuis l'hélice d'occlusion **(3).**

19. Dispositif selon l'une des revendications 1 à 18, **caractérisé en ce que** sur le moyen d'immobilisation **(10)** sont fondus différents fils polymères **(13)** plus courts, dont les extrémités sortent vers l'extérieur depuis l'hélice d'occlusion **(3).**

20. Dispositif selon l'une des revendications 10 à 19, **caractérisé en ce que** les fils polymères **(10)** sont composés de fibres individuelles.

21. Dispositif selon la revendication 20, **caractérisé en ce que** certaines fibres **(14)** du fil polymère **(10)** sortent vers l'extérieur depuis l'hélice d'occlusion **(3),** tandis que d'autres fibres du fil polymère **(10)** traversent l'ouverture **(9)** de l'hélice d'occlusion **(3)** sur toute sa longueur.

22. Dispositif selon la revendication 20 ou 21, **caractérisé en ce que** certaines fibres **(14)** du fil polymère **(10)** sont plus courtes que le fil polymère **(10)** dans son ensemble et leurs extrémités sortent vers l'extérieur depuis l'hélice d'occlusion **(3).**

23. Dispositif selon l'une des revendications 1 à 22, **caractérisé en ce que** certaines fibres sont collées ou fondues sur le moyen d'immobilisation **(10)** et sortent vers l'extérieur depuis l'hélice d'occlusion **(3).**

24. Dispositif selon l'une des revendications 1 à 23, **caractérisé en ce que** l'hélice d'occlusion **(3)** présente plusieurs endroits **(6)** corrodables électrolytiquement, un moyen d'immobilisation **(10)** étant placé dans chaque segment de l'hélice d'occlusion **(3)** situé entre ces endroits **(6).**

25. Hélice d'occlusion destinée à une utilisation en liaison avec le dispositif selon l'une des revendications 1 à 24.
